# EUROPEAN PATENT SPECIFICATION

(11) **EP 0 405 883 B1**
(45) Date of publication and mention of the grant of the patent: **11.10.1995**
(21) Application number: 90306919.3
(22) Date of filing: 25.06.1990
(51) Int. Cl.: A61B 17/34

(54) **A Pneumoperitoneal needle**
Pneumoperitoneum-Nadel
Aiguille pneumopéritonéale

(30) Priority: 27.06.1989 GB 8914728
(43) Date of publication of application: 02.01.1991
(73) Proprietor: Rocket of London Limited, Watford, Hertfordshire WD2 4XX (GB)
(72) Inventor: Robson, Richard, Washington, Tyne & Wear NE38 0PU (GB); Meredith, Hadyn, Lymington, Hampshire (GB)
(74) Representative: Pattullo, Norman

(56) References cited:
- EP-A- 0 153 190
- EP-A- 0 339 945
- US-A- 1 527 291
- US-A- 2 642 873
- US-A- 3 840 008
- US-A- 4 808 168

## Description

The invention relates to pneumoperitoneal needles and in particular, pneumoperitoneal needles of the Veress type.

Pneumoperitoneal needles of the Veress type are generally used to facilitate endoscopic examination of the abdominal cavity. The needle is inserted through the external wall of the abdominal cavity which is then insufflated with a suitable gas through the needle before the endoscope is inserted. The Veress type pneumoperitoneal needles have a spring loaded blunt obturator which is mounted inside a larger diameter hollow needle. During insertion of the needle through the abdominal wall the resistance against the end of the pneumperitoneal needle is sufficient to force the obturator to retract within the hollow needle which enables the sharp tip of the needle to penetrate the abdominal wall. Once the pneumoperitoneal needle has penetrated the abdominal wall and enters the abdominal cavity the resistance against the blunt end of the obturator decreases sufficiently to allow the spring to push the blunt end of the obturator forward so that it extends beyond the sharp tip of the needle. This helps to prevent puncture or laceration of the intra-abdominal structures by the sharp tip of the needle.

Traditionally, Veress type pneumoperitoneal needles have been manufactured from precision machined stainless steel parts and are therefore expensive. Hence, they are intended for multiple re-use and must be cleaned and sterilized between uses and sharpened regularly.

Recently, attempts have been made to produce disposable, one time use only Veress type pneumoperitoneal needles. However, mass production of a relatively inexpensive disposable needle has been difficult because of the need to prevent relative rotation of the obturator within the hollow needle. This is necessary to prevent the obturator aperture, through which fluid is transferred, from being masked by the hollow needle, which occurs if the obturator rotates.

US Patent No 1,527,291 discloses a surgical needle having an obturator which has several lateral openings near one end. The end of the obturator can protrude from the tip of the needle.

Another problem with conventional pneumoperitoneal needles is that it is difficult to determine when the needle has passed through the abdominal wall. If an operator attempts to insufflate the abdominal cavity before the end of the needle has entered the abdominal cavity damage to the abdominal wall can result.

In accordance with the present invention there is provided a pneumoperitoneal needle comprising a hollow needle for penetrating a body cavity, a hollow obturator having a blunt tip and through which a fluid may be transferred to or from the body cavity, the obturator being mounted within the needle and linearly movable between a first position in which the blunt tip of the obturator extends beyond the tip of the needle and a second position in which the tip of the needle extends beyond the tip of the obturator, and biassing means to bias the obturator to the first position, the biassing means being such that during penetration of the body cavity the resistance is sufficient to overcome the biassing means and move the obturator from the first position to the second position and such that after the body cavity has been penetrated the obturator returns to the first position under the action of the biassing means, and wherein the obturator is rotatable within the needle and has through its wall at least two apertures adjacent to its tip, such that when the obturator is in the first position fluid may pass through at least one of the apertures and at least one of the apertures can be at least partially masked by the tip of the needle, characterised in that the apertures are so positioned on the obturator that when the obturator is in the first position the area of aperture through which fluid may pass remains substantially independent of the rotational position of the obturator relative to the needle.

Thus a Veress type pneumoperitoneal needle can be produced which does not require rotation of the obturator within the hollow needle to be prevented. This enables the pneumoperitoneal needle to be less complex in design and hence simplifies the manufacture of the pneumoperitoneal needle.

Preferably the two apertures are situated diametrically opposite each other on the obturator and are laterally offset from each other along the length of the obturator.

Typically, the pneumoperitoneal needle has a handle having a central bore and the fluid is passed through the bore of the handle to or from a fluid transfer means which is connected to the handle.

Preferably, the fluid transfer means comprises a valve to enable the transfer of fluid to be regulated. Typically, the fluid transfer means is removably connected to the handle to enable the obturator to be removed from the hollow needle through the central bore.

In the preferred embodiment the biassing means is a helical spring mounted in the handle between the obturator and the fluid transfer means.

Preferably, the pneumoperitoneal needle is of the Veress type and is designed to be inserted into the abdominal cavity and typically the fluid is a gas which is to be transferred into the abdominal cavity to insufflate the abdominal cavity.

An example of a pneumoperitoneal needle according to the invention will now be described with reference to the accompanying drawings, in which:-
Typically, the handle is manufactured from clear plastics material and the surface adjacent to the viewing aperture or window is textured so that the coded member is substantially visible only through the viewing aperture or window which is not textured.

The handle may comprise two apertures, one aperture on either side of the handle.

Typically, the handle has a central bore and the fluid is passed through the bore of the handle to or from a fluid transfer means which is connected to the handle.

Preferably, the fluid transfer means comprises a valve to enable the transfer of fluid to he regulated. Typically, the fluid transfer means is removably conected to the handle to enable the obturator to be removed from the hollow needle through the central bore.

In the preferred embodiment the biassing means is a helical spring mounted in the handle between the obturator and the fluid transfer means and preferably the coded member is located between the obturator and the biassing means. However, the coded member could be located on the external surface of the obturator.

Preferably, the pneumoperitoneal needle is of the Verres type and is designed to be inserted into the abdominal cavity and typically the fluid is a gas which is to be transferred into the abdominal cavity to insufflate the abdominal cavity.

An example of a pneumoperitoneal needle according to the invention will now be described with reference to the accompanying drawings, in which:-
Figure 1 is a plan view of a Veress type pneumoperitoneal needle;
Figure 2 is a side view of the pneumoperitoneal needle shown in Figure 1 with a partial section on the line B-B;
Figure 3 shows an obturator for use in the pneumoperitoneal needle shown in Figures 1 and 2; and,
Figures 4a and 4b show in detail an indicator for use in the pneumoperitoneal needle shown in Figures 1 and 2.

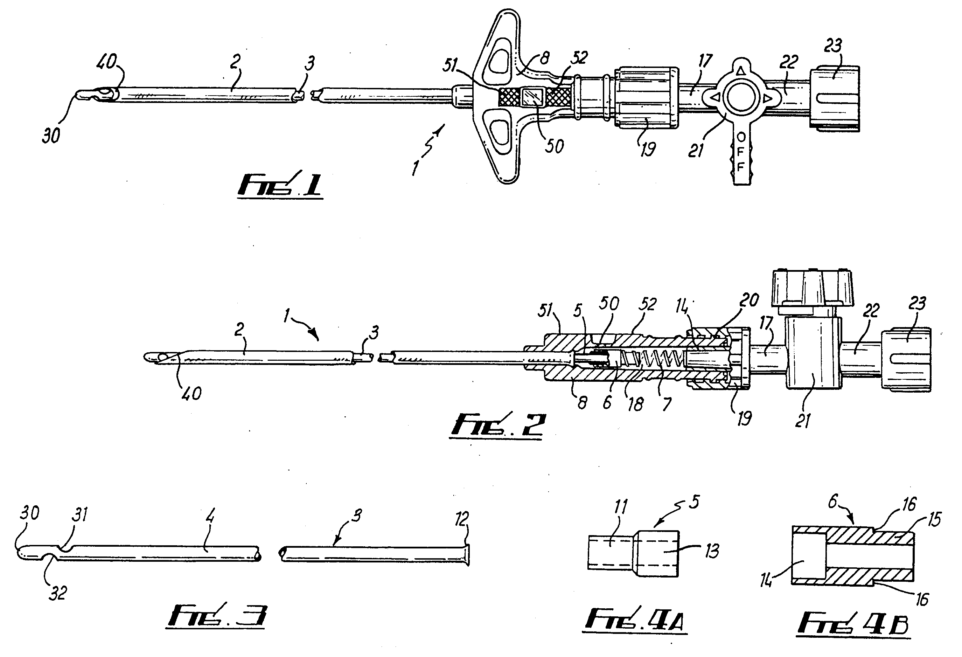

Figure 1 shows a pneumoperitoneal needle of the Veress type which is designed to be inserted through the abdominal wall in order to penetrate the abdominal cavity to enable the abdominal cavity to be insufflated with a suitable gas. The pneumoperitoneal needle 1 comprises a hollow needle 2 which is mounted in a bore 18 of a moulded plastic handle 8. An obturator 3 is mounted within the hollow needle 2 and the plastic handle 8 (see Figure 2) by inserting it through the bore 18. The obturator 3 may slide freely within the hollow needle 2 and bore 18.

The obturator 3 is shown in more detail in Figure 3 and is constructed from a hollow metal tube 4. At one end 30 the hollow metal tube 4 of the obturator 3 is formed into a smooth hemi-sphere and at the other end 12 the hollow tube 4 of the obturator 3 is flared. The end 12 of the obturator 3 is flared to prevent the obturator sliding completely through the hollow needle 2 and it also provides a mounting for section 5 of an indicator. Adjacent to the end 30 are two apertures 31,32 in the tube 4. The apertures 31, 32 are formed so that they are diametrically opposite each other and so that they are also laterally offset from each other.

The apertures 31, 32 are arranged so that irrespective of the rotational orientation of the obturator 3 within the hollow needle 2 there is always a substantially constant aperture area which is exposed and is not masked by the hollow needle 2 when the obturator 3 is in its extended position. This is achieved by positioning the apertures 31, 32 such that when one aperture is masked by the hollow needle 2 the other aperture is completely exposed. When the obturator 3 is in a position whereby half of one aperture is masked by the needle 2 simultaneously half of the other aperture is also masked by the hollow needle 2. In this manner irrespective of the rotational orientation of the obturator 3 within the hollow needle 2 the total aperture area which is exposed is generally equal to the area of one aperture.

The hollow needle 2 has a sharpened tip 40 with a standard angled hypodermic point. The hypodermic point 40 enables the pneumoperitoneal needle to be inserted through the abdominal wall into the abdominal cavity in the same manner that a conventional needle would be inserted.

Abutting the chamfered or flared end 12 of the obturator 3 is the indicator which comprises two sections 5, 6. The sections 5, 6 are shown in more detail in Figures 4a and 4b respectively. The section 5 of the indicator is typically coloured red and the section 6 is typically coloured green. However, the sections 5, 6 could be coded with any other suitable colours or even have writing on their surfaces in order to distinguish one section from the other. As shown in Fig. 2 the end 11 of the section 5 is designed to fit into the chamfered end 12 of the obturator 3 but has a diameter which is greater than the internal diameter of the hollow tube 4 of the obtruator 3. The other end 13 of the section 5 is designed to be inserted into a hollow 14 in the section 6. The other end 15 of the section 6 is dimensioned so that it fits inside a helical spring 7 so that the helical spring butts against a step 16 in the section 6.

The other end of the helical spring 7 abuts against a hollow tube 17 inserted into the bore 18 in the handle 8 and is held in the bore 18 against the action of the spring 7 by means of a collar 19 which has an internal thread so that it may be screwed onto the outside of the handle 8 on a thread 20. Connected to the hollow tube 17 is a valve 21 which may be opened to permit the gas to be transferred into the abdominal cavity or may be closed to prevent the gas being transferred into the abdominal cavity. In the position shown in Figures 1 and 2 the valve 21 is in the open position. On the opposite side of the valve 21 from the tube 16 is another tube 22 with a threaded collar 23 in order to enable the pneumoperitoneal needle 1 to be connected to insufflating apparatus (not shown) to enable the abdominal cavity to be insufflated.

In use, the valve 21 is turned to the closed position and the collar 23 and tube 22 are connected to the insufflating apparatus. An operator such as a surgeon then presses the hemi-spherical end 30 of the obturator 3 against the external skin of the abdominal wall of a patient by holding the pneumoperitoneal needle 1 by the handle 8. As the pneumoperitoneal needle 1 is pressed against the external surface of the skin the resistance of the skin causes the obturator 3 to be pushed against the spring 7 so that the spring 7 compresses and the obturator is retracted into the pnuemoneedle 1 so that the hypodermic point 40 of the hollow needle 2 is exposed. When this happens the pressure exerted by the operator is sufficient to enable the hypodermic point 40 of the hollow needle 2 to penetrate the external skin of the abdominal wall so that the pneumoperitoneal needle 1 penetrates the abdominal wall and enters the addominal cavity. As the pneumoperitoneal needle 1 penetrates the abdominal cavity the resistance exerted by the abdominal wall is removed from the end 30 of the obturator and the spring 7 forces the obturator 3 back to its extended position by means of the indicating sections 5, 6.

The smooth hemi-spherical surface of the end 30 of the obturator 3 now extends beyond the sharp hypodermic point 40 of the hollow needle 2 and this helps to prevent damage to the structures inside the abdominal cavity, such as the large intestine by laceration or puncture by the hypodermic point 40 of the hollow needle 2.

In order to insufflate the abdominal cavity the insufflating apparatus connected to the tube 22 by the collar 23 is switched on and the valve 21 is turned to the open position to allow the gas to flow through the tube 19, the spring 7 and the indicating sections 5, 6 into the obturator 3. The gas flows through the inside of the obturator 3 and exits the obturator 3 via the apertures 31, 32.

Because the area of the apertures 31, 32 which are exposed and are not masked by the hollow needle 2 is always substantially constant, as described above, there is always a constant pressure of gas exiting through the apertures 31, 32. Hence, it is not necessary to keep the obturator 3 in the same relative rotational orientation with respect to the hollow needle 2 so that the gas pressure and hence the voulume of gas exiting throught the aperture area is always constant.

This represents a significant advance over prior designs of obturators where there was only one aperture and therefore it was necessary to always maintain the obturator 3 in the same relative orientation to the hollow of needle 2 to prevent rotation of the obturator 3 causing the needle 2 to mask a portion or all of the aperture. Hence the provision of two apertures enables the manufacturing of the obturator and other components to be simplified by not requiring a keying portion or another fixture on the obturator 3 to prevent the obturator 3 rotating.

When the obturator 3 is in the extended position as shown in Figure 1 only the green section 6 of the indicator is visible through a window 50 in the handle 8. The window 50 is shaped to form a lens which magnifies the objects within the handle. However, if the obturator 3 is not in its extended position but is in the retracted or a partially retracted position, as is the case when the pneumoperitoneal needle 1 is penetrating the abdominal wall, at least a portion of the red section 5 of the indicator is visible through the window 50. In this example the window 50 is highly polished to enhance its optical quality and regions 51, 52 of the handle 8 which are immediately adjacent to the window 50 are textured to reduce the optical quality of the clear plastic handle so that the indicator is not easily visible through these sections 51, 52. Hence, an operator can clearly tell whether the obturator 3 is fully extended and therefore the operator knows when the pneumoperitoneal needle has entered the abdominal cavity.

In this example the obturator 3 and the hollow needle 2 are manufactured from stainless steel and the spring 7 is also stainless steel. The indicating sections 5, 6 are high density polyethylene and the handle 8 is injection moulded from a clear high molecular weight styrene. This method of manufacture enables a cheap pneumoperitoneal needle to be produced in high quantities. Hence, it is possible to produce a one time use only pneumoperitoneal needle which obviates the need for the pneumoperitoneal needles to be cleaned and sterilized after each use and for the regular sharpening that is required with conventional needles.

## Claims

1. A pneumoperitoneal needle comprising a hollow needle (2) for penetrating a body cavity, a hollow obturator (3) having a blunt tip (30) and through which a fluid may be transferred to or from the body cavity, the obturator (3) being mounted within the needle (2) and linearly movable between a first position in which the blunt tip (30) of the obturator extends beyond the tip (40) of the needle (2) and a second position in which the tip (40) of the needle (2) extends beyond the tip (30) of the obturator (3), and biassing means (7) to bias the obturator (3) to the first position, the biassing means (7) being such that during penetration of the body cavity the resistance is sufficient to overcome the biassing means (7) and move the obturator (3) from the first position to the second position and such that after the body cavity has been penetrated the obturator (3) returns to the first position under the action of the biassing means (7), and wherein the obturator (3) is rotatable within the needle (2) and has through its wall at least two apertures (31, 32) adjacent to its tip (30) such that when the obturator (3) is in the first position fluid may pass through at least one of the apertures (31, 32) and at least one of the apertures (31, 32) can be at least partially masked by the tip of the needle (2), characterised in that the apertures (31, 32) are so positioned on the obturator (3) that when the obturator (3) is in the first position the area of aperture through which fluid may pass remains substantially independent of the rotational position of the obturator (3) relative to the needle (2).

2. A pneumoperitoneal needle according to Claim 1, characterised in that there are two apertures (31, 32) positioned diametrically opposite each other on the obturator.

3. A pneumoperitoneal needle according to Claim 1 or Claim 2 characterised in that there are two apertures (31, 32) which are laterally off-set from each other along the length of the obturator (3).

4. A pneumoperitoneal needle according to any one of the preceding Claims, characterised in that the biassing means comprises a helical spring.

## Patentansprüche

1. Pneumoperitoneale Nadel, bestehend aus einer hohlen Nadel (2) zum Durchdringen eines Körperhohlraumes, einem hohlen Absperrorgan (3) mit einer stumpfen Spitze (30) und durch welches hindurch ein Fluid zu und von dem Körperhohlraum übertragen werden kann, wobei das Absperrorgan (3) innerhalb der Nadel (2) angeordnet und linear beweglich ist zwischen einer ersten Position, in welcher die stumpfe Spitze (30) des Absperrorgans über die Spitze (40) der Nadel (2) hinaus verläuft, und einer zweiten Position, in welcher die Spitze (40) der Nadel (2) über die Spitze (30) des Absperrorgans (3) hinaus verläuft, und einer Vorspanneinrichtung (7) zum Vorspannen des Absperrorgans (3) in die erste Position, wobei die Vorspanneinrichtung (7) derart ist, daß während des Durchdringens des Körperhohlraumes der Widerstand ausreicht, um die Vorspanneinrichtung (7) zu überwinden und das Absperrorgan (3) aus der ersten Positon in die zweite Position zu bewegen, sowie derart, daß nach dem Durchdringen des Körperhohlraumes das Absperrorgan (3) in die erste Position unter der Wirkung der Vorspanneinrichtung (7) zurückkehrt, und wobei das Absperrorgan (3) innerhalb der Nadel (2) drehbar ist und wenigstens zwei Öffnungen (31, 32) durch seine Wand hindurch neben der Spitze (30) aufweist, sodaß, wenn das Absperrorgan (3) in der ersten Position ist, Fluid durch wenigstens eine der Öffnungen (31, 32) hindurchgehen kann und wenigstens eine der Öffnungen (31, 32) wenigstens teilweise durch die Spitze der Nadel (2) verdeckt werden kann, dadurch gekennzeichnet, daß die Öffnungen (31, 32) so an dem Absperrorgan (3) positioniert sind, daß, wenn das Absperrorgan (3) in der ersten Position ist, der Bereich der Öffnung, durch welche das Fluid hindurchgehen kann, im wesentlichen unabhängig von der Drehposition des Absperrorgans (3) relativ zu der Nadel (2) verbleibt.

2. Pneumoperitoneale Nadel nach Anspruch 1, dadurch gekennzeichnet, daß zwei Öffnungen (31, 32) vorhanden sind, die an dem Absperrorgan zueinander diametral entgegengesetzt sind.

3. Pneumoperitoneale Nadel nach Anspruch 1 oder Anspruch 2, dadurch gekennzeichnet, daß zwei Öffnungen (31, 32) vorhanden sind, die entlang der Länge des Absperrorgans (3) zueinander seitlich versetzt sind.

4. Pneumoperitoneale Nadel nach einem der vorhergehenden Ansprüche, dadurch gekennzeichnet, daß die Vorspanneinrichtung eine Spiralfeder umfaßt.

## Revendications

1. Aiguille pneumopéritonéale comprenant une aiguille creuse (2) pour pénétrer dans une cavité d'un corps, un obturateur creux (3) ayant un bout émoussé (30) et à travers lequel on peut transférer un fluide dans et hors de la cavité du corps, l'obturateur (3) étant monté à l'intérieur de l'aiguille (2) et étant déplaçable linéairement entre une première position dans laquelle le bout émoussé (30) de l'obturateur s'étend au-delà du bout (40) de l'aiguille (2) et une seconde position dans laquelle le bout (40) de l'aiguille (2) s'étend au-delà du bout (30) de l'obturateur (3), et un moyen de décalage (7) pour décaler l'obturateur (3) vers la première position, le moyen de décalage (7) étant tel que durant la pénétration de la cavité du corps la résistance est suffisante pour vaincre le moyen de décalage (7) et déplacer l'obturateur (3) depuis la première position jusqu'à la seconde position et tel que après que la cavité du corps a été pénétrée l'obturateur (3) retourne à la première position sous l'action du moyen de décalage (7), et dans laquelle l'obturateur (3) peut être pivoté à l'intérieur de l'aiguille (2) et comprend dans sa paroi au moins deux ouvertures (31, 32) adjacentes à son bout (30) de telle façon que lorsque l'obturateur (3) est dans sa première position du fluide peut passer à travers au moins l'une des ouvertures (31, 32) et qu'au moins l'une des ouvertures (31, 32) peut être au moins partiellement masquée par le bout de l'aiguille (2), caractérisée en ce que les ouvertures (31, 32) sont positionnées sur l'obturateur (3) de telle façon que lorsque l'obturateur (3) est dans la première position la zone d'ouverture à travers laquelle le fluide peut passer reste sensiblement indépendante de la position de rotation de l'obturateur (3) par rapport à l'aiguille (2).

2. Aiguille pneumopéritonéale selon la revendication 1, caractérisée en ce qu'il y a deux ouvertures (31, 32) positionnées de façon diamétralement opposée l'une par rapport à l'autre sur l'obturateur.

3. Aiguille pneumopéritonéale selon la revendication 1 ou la revendication 2, caractérisée en ce qu'il y a deux ouvertures (31, 32) qui sont latéralement décalées l'une par rapport à l'autre dans le sens de la longueur de l'obturateur (3).

4. Aiguille pneumopéritonéale selon l'une quelconque des revendications précédentes, caractérisée en ce que le moyen de décalage comprend un ressort hélicoïdal.
